# EUROPEAN PATENT APPLICATION

(11) **EP 2 302 587 A1**
(43) Date of publication of application: **30.03.2011**
(21) Application number: 10171357.6
(22) Date of filing: 30.07.2010
(51) Int. Cl.: G06T 7/00

(54) **Ultrasound system and method of measuring fetal rib**

(30) Priority: 20.08.2009 KR 20090077096
(71) Applicant: MEDISON CO., LTD., Gangwon-do 250-875 (KR)
(72) Inventor: Kim, Sung Hee, 135-782 Seoul (KR); Kim, Jung, 137-063 Seoul (KR); Kim, Jeong-Sik, 467-050 Gyeonggi-Do (KR)
(74) Representative: Schmid, Wolfgang

(57) **Abstract**

Disclosed are an ultrasound system of measuring a number of ribs of a fetus and a method thereof. The ultrasound system of measuring the number of ribs of the fetus in three dimensions may include a setting unit that is used by a user, to set a portion where the number of ribs is measured in an ultrasound image, as a region of interest (ROI), a measuring unit to measure the number of ribs in the set ROI by using an auto rib extracting algorithm, and a display unit to display the measured number of ribs.

## Description

### CROSS-REFERENCE TO RELATED APPLICATION

This application claims the benefit of Korean Patent Application No. 10-2009-0077096, filed on August 20, 2009, in the Korean Intellectual Property Office, the disclosure of which is incorporated herein by reference.

### BACKGROUND

### 1. Field

The present invention relates to an ultrasound system and method of measuring a number of ribs of a fetus, and more particularly, to an ultrasound system and method of setting a portion where the number of ribs is measured as a region of interest (ROI), measuring the number of ribs in the set ROI, and displaying the measured number of ribs.

### 2. Description of the Related Art

Generally, an ultrasound system may transmit an ultrasound signal toward a predetermined portion inside a body from a surface of the body, and may obtain an image about a section of a soft tissue or a bloodstream by using information of the ultrasound signal having been reflected from a tissue inside the body.

The ultrasound system has an advantage of being small, inexpensive, reliable, and not exposing a subject to an X-ray and the like, and thus, the ultrasound system is commonly used together with other image diagnostic devices, such as a computerized tomography (CT) scanner, a magnetic resonance image (MRI) device, a nuclear medicine device, and the like. Particularly, the ultrasound system may display an image of inside the body in real time, thereby being applicable for various uses.

As a utilization field of the ultrasound system is expanded, there are various demands on an ultrasound image of the ultrasound system. In particular, a lesion or a tissue of a patient may need to be precisely observed when a procedure, such as a medical checkup, a biopsy, an operation, and the like, is performed. Accordingly, the ultrasound system may need to obtain a three-dimensional (3D) ultrasound image.

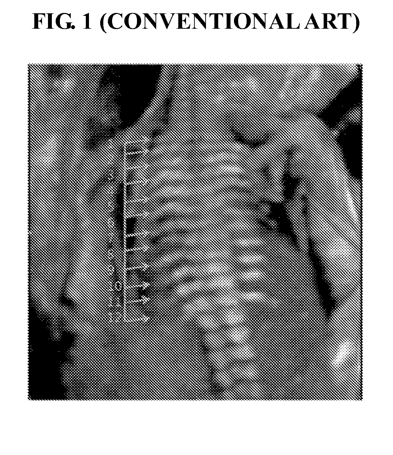

A number of ribs of a fetus is generally twelve in a 3D ultrasound image as illustrated in FIG. 1. However, when the number of ribs is eleven, there is a higher probability that the fetus has Down's syndrome. Accordingly, the number of ribs of the fetus is an important index for checking a probability of a disorder in the fetus.

A conventional system may have inconvenience in that a user calculates the number of ribs of the fetus by hand from a rendered 3D image as illustrated in FIG. 1.

### SUMMARY

An aspect of the present invention provides an ultrasound system and method that accurately measures a number of ribs of a fetus in a three-dimensional (3D) ultrasound image.

Another aspect of the present invention provides an ultrasound system and method that displays a rib area and a number of ribs extracted from a 3D ultrasound image.

According to an aspect of the present invention, there is provided an ultrasound system of measuring a number ribs of a fetus, the ultrasound system includes a setting unit to set, by a user, a portion where the number of ribs is measured as a region of interest (ROI) in an ultrasound image, a measuring unit to measure the number of ribs in the set ROI, by using an auto rib extracting algorithm, and a display unit to display the measured number of ribs.

The measuring unit may enhance a rib image in the ROI, may generate a binary image by extracting an area having a brightness greater than or equal to a reference value from the enhanced rib image, may classify discrete areas from the binary image, may connect the classified discrete areas along a principle axis to generate rib areas, and may count a number of the rib areas to measure the number of ribs.

The measuring unit may enhance a picture quality or a definition of the rib image through a rib enhancement filtering process for segmentation of a ROI image.

The display unit may display the measured rib area and the number of ribs.

According to another aspect of the present invention, there is provided a method of measuring a number of ribs in an ultrasound system including a setting unit, a measuring unit, and a display unit, the method including setting, by a user, an ROI to measure, through the setting unit, the number of ribs in an ultrasound area, measuring, by the measuring unit, the number of ribs in the ROI, based on an auto rib extracting algorithm, and displaying, by the display unit, information about the measured number of ribs.

Additional aspects and/or advantages will be set forth in part in the description which follows and, in part, will be apparent from the description, or may be learned by practice of the embodiments.

### EFFECT

According to the present invention, there is no need for counting a number of ribs by hand, since the number of ribs is automatically counted from a 3D ultrasound image, and thus, a user's convenience increases.

According to the present invention, there is provided an effect of easily obtaining a rib area and a number of ribs extracted from a 3D ultrasound image.

### BRIEF DESCRIPTION OF THE DRAWINGS

These and/or other aspects and advantages will become apparent and more readily appreciated from the following description of the embodiments, taken in conjunction with the accompanying drawings of which:
- FIG. 1: is a diagram illustrating a number of ribs of a normal fetus in a three dimensional (3D) ultrasound image, according to a conventional art;
- FIG. 2: is a diagram illustrating a format of an ultrasound system of measuring a number of ribs of a fetus according to an exemplary embodiment of the present invention;
- FIG. 3: is a diagram illustrating an example where a region of interest (RIO) is set to measure a number of ribs according to an exemplary embodiment of the present invention;
- FIG. 4: is a diagram illustrating an example of a result from each operation of an auto rib extracting algorithm according to an exemplary embodiment of the present invention;
- FIG. 5: is a diagram illustrating an example of displaying a measured rib area and a number of ribs according to an exemplary embodiment of the present invention;
- FIG. 6: is a flowchart illustrating a method of measuring a number of ribs of a fetus according to an exemplary embodiment of the present invention; and
- FIG. 7: is a diagram illustrating a detailed procedure of measuring a number of ribs of a fetus according to an exemplary embodiment of the present invention.

### DETAILED DESCRIPTION

Reference will now be made in detail to example embodiments, examples of which are illustrated in the accompanying drawings, wherein like reference numerals refer to the like elements throughout. An ultrasound diagnosis apparatus utilizing a touch interaction is described below to explain the present disclosure by referring to the figures.

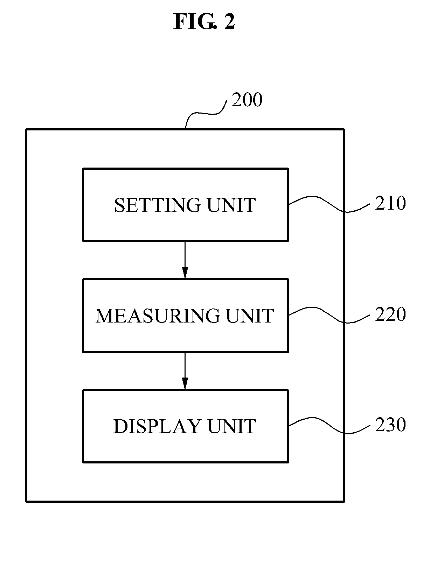

FIG. 2 illustrates a format of an ultrasound system 200 of measuring a number of ribs of a fetus according to an exemplary embodiment of the present invention.

Referring to FIG. 2, the ultrasound system 200 that measures the number of ribs of the fetus according to an embodiment of the present invention may include a setting unit 210, a measuring unit 220, and a display unit 230. A user sets, through the setting unit 210, a portion where the number of ribs is measured in an ultrasound image, as a region of interest (ROI).

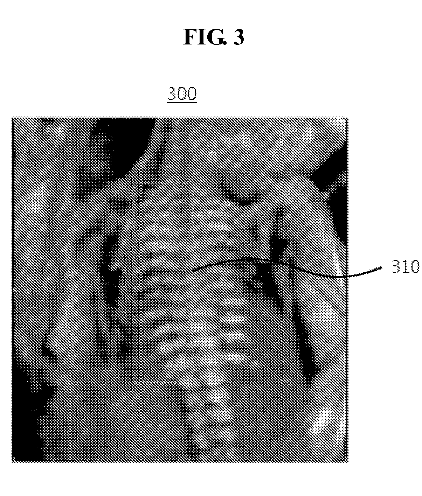

FIG. 3 illustrates an example where a RIO 310 is set to measure a number of ribs according to an exemplary embodiment of the present invention;

Referring to FIG. 3, a user may set a portion where the number of ribs is measured in a 3D ultrasound image 300 as an ROI 310, through the setting unit 210.

The measuring unit 220 may measure the number of ribs in the set ROI 310 based on an auto rib extracting algorithm. In other words, the measuring unit 220 may enhance a rib image in the ROI 310, may generate a binary image by extracting an area having a brightness greater than or equal to a reference value from the enhanced rib image, may classify discrete areas from the binary image, may connect the classified discrete areas along a principle axis to generate rib areas, and may count a number of the rib areas to measure the number of ribs.

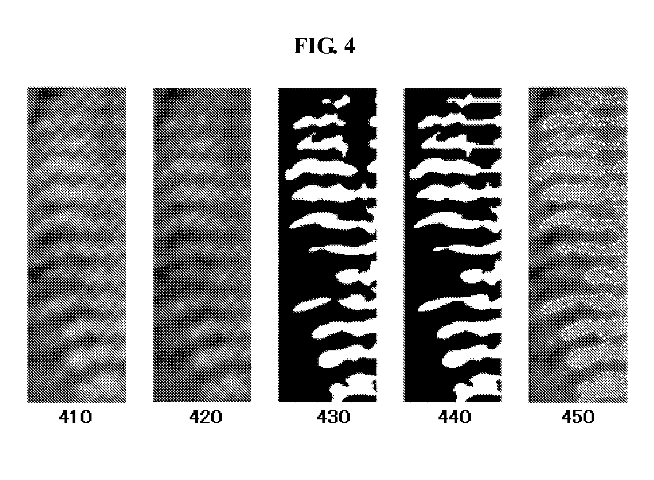

FIG. 4 illustrates an example of a result from each operation of an auto rib extracting algorithm according to an exemplary embodiment of the present invention.

Referring to FIG. 4, the measuring unit 220 may generate an enhanced image 420 by performing a rib enhancement filtering process for segmentation of an ROI image 410, as a preprocessing with respect to the ROI image 410. The enhanced image 420 is a 3D ultrasound image of which a picture quality or a definition is enhanced to make a rib to be easily observed in the ROI image 410. That is, the measuring unit 220 may enhance a picture quality or a definition of a rib image through the rib enhancement filtering process for segmentation of the ROI image 410. As an example, the measuring unit 220 may perform the rib enhancement filtering process with respect to the ROI image 410 through an image enhancement process using various schemes, such as a smoothing scheme, a sharpening scheme, and the like. Also, the measuring unit 220 may generate a binary image by extracting an area having a brightness greater than or equal to a reference value from the enhanced rib image 420. Also, the measuring unit 220 may generate a discrete image 430 by classifying discrete images from the binary image. Also, the measuring unit 220 may generate a rib area image 440 by connecting the classified discrete areas along a principle axis and generating rib areas. Also, the measuring unit 220 may count a number of the rib areas, thereby generating a result image 450 indicating a result of the number of ribs.

The display unit 230 may display the measured number of ribs.

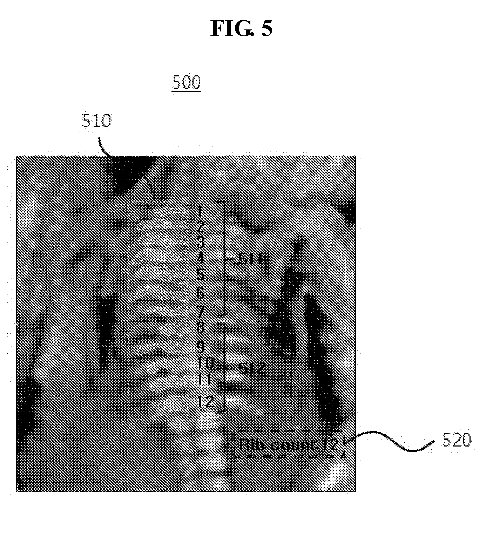

FIG. 5 illustrates an example of displaying a measured rib area and a number of ribs according to an exemplary embodiment of the present invention.

Referring to FIG. 5, the display unit 230 may display a measured rib area 510 and a number of ribs 520 in a 3D ultrasound image 500. As an example, the measured rib area 510 may be classified into true ribs 511 and false ribs 521.

As described above, the ultrasound system 200 may set a portion where a number of ribs is measured in a 3D ultrasound image, as an ROI, may measure a number ribs in the set ROI, and may display the measured number of ribs.

Accordingly, the ultrasound system 200 may display the extracted rib area and the number of ribs, and may alleviate the inconvenience of counting the number of ribs, which is one of important indexes, by hand, thereby increasing a user's convenience.

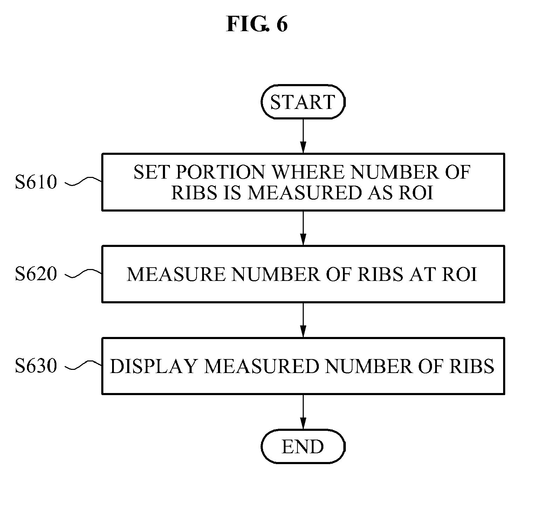

FIG. 6 is a flowchart illustrating a method of measuring a number of ribs of a fetus according to an exemplary embodiment of the present invention.

Referring to FIGS. 2 to 6, in operation S610, a user sets an ROI where a number of ribs of a fetus is measured in an ultrasound image, through the setting unit 210. As an example, the user may set a portion where the number of ribs is measured in the 3D ultrasound image 300 as the ROI 310.

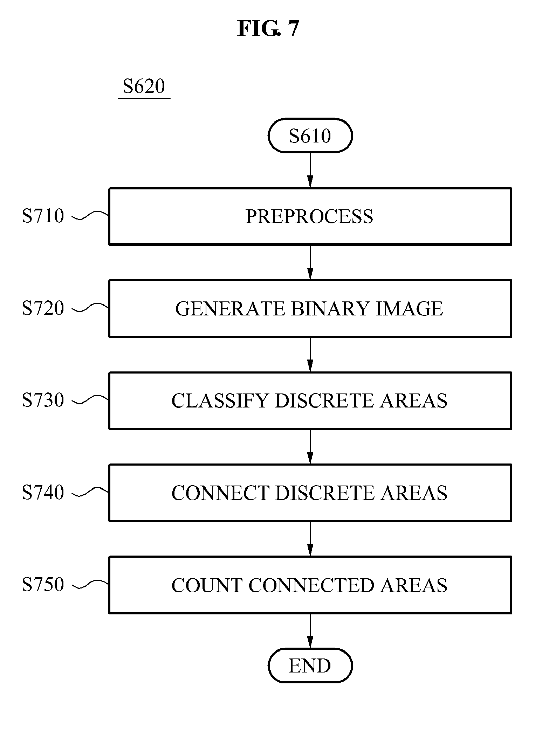

In operation S620, the measuring unit 220 measures the number of ribs in the ROI based on an auto rib extracting algorithm. Hereinafter, referring to FIG. 7, the measuring of the number of ribs of the fetus based on the auto rib extracting algorithm, will be described in detail.

FIG. 7 illustrates a detailed procedure of measuring a number of ribs of a fetus according to an exemplary embodiment of the present invention.

Referring to FIG. 7, in operation S710, the measuring unit 220 performs a preprocessing to enhance a rib image in the ROI. That is, in operation S710, the measuring unit 220 may enhance a picture quality or a definition of a 3D ultrasound image to make the rib image included in the ROI to be clearly shown, through the rib enhancement filtering process for segmentation of the ROI image. As an example, in operation S720, the measuring unit 220 generates an enhanced image 420 that is a 3D ultrasound image of which a picture quality or a definition of is enhanced, as a result of performing the preprocessing on the ROI image 410, the preprocessing being the image enhancement process using various schemes, such as a smoothing scheme, a sharpening scheme, and the like.

In operation S720, the measuring unit 220 generates a binary image by extracting an area having a brightness greater than or equal to a reference value in the enhanced rib image.

In operation S730, the measuring unit 220 classifies discrete areas based on levels of areas in the binary images. That is, in operation S730, the measuring unit 220 generates the discrete area image 430 by classifying the discrete areas from the binary image.

In operation S740, the measuring unit 220 connects the classified discrete areas along a principle axis to generate rib areas. That is, in operation S740, the measuring unit 220 generates a rib area image 440 by linking discontinuous areas of the classified areas along the principle axis.

In operation S750, the measuring unit 220 measures information about the number of ribs by counting a number of the rib areas. As an example, in operation S750, the measuring unit 220 measures the number of ribs by counting the rib areas in the result image 450.

In operation S630, the display unit 230 displays information about the measured number of ribs. As an example, in operation S630, the display unit 230 displays the extracted rib area 510 and the number of ribs 520 extracted from the 3D ultrasound image 300, as the information about the measured number of ribs.

Accordingly, in the ultrasound system according to an embodiment of the present invention, a method of measuring a number of ribs may set a portion where the number of ribs is measured in a 3D ultrasound image as an ROI, may automatically measure the number of ribs in the set ROI, and may display the measured the number of ribs, thereby removing the inconvenience of counting the number of ribs, which is one of important indexes, by hand. Therefore, user's convenience may increase.

A method of measuring a number of ribs of a fetus according to the above-described exemplary embodiments of the present invention may be recorded in computer-readable media including program instructions to implement various operations embodied by a computer. The media may also include, alone or in combination with the program instructions, data files, data structures, and the like. Examples of computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few example embodiments have been shown and described, it would be appreciated by those skilled in the art that changes may be made in these example embodiments without departing from the principles and spirit of the invention, the scope of which is defined in the claims and their equivalents.

## Claims

1. An ultrasound system of measuring a number ribs of a fetus, the ultrasound system comprising:
a setting unit to set, by a user, a portion where the number of ribs is measured as a region of interest (ROI) in an ultrasound image;
a measuring unit to measure the number of ribs in the set ROI, by using an auto rib extracting algorithm; and
a display unit to display the measured number of ribs.

2. The ultrasound system of claim 1, wherein the measuring unit enhances a rib image in the ROI, generates a binary image by extracting an area having a brightness greater than or equal to a reference value from the enhanced rib image, classifies discrete areas from the binary image, connects the classified discrete areas along a principle axis to generate rib areas, and counts a number of the rib areas to measure the number of ribs.

3. The ultrasound system of claim 2, wherein the measuring unit enhances a picture quality or a definition of the rib image through a rib enhancement filtering process for segmentation of a ROI image.

4. The ultrasound system of claim 1, wherein the display unit displays the measured rib area and the number of ribs.

5. A method of measuring a number of ribs in an ultrasound system including a setting unit, a measuring unit, and a display unit, the method comprising:
setting, by a user, an ROI to measure, through the setting unit, the number of ribs in an ultrasound area;
measuring, by the measuring unit, the number of ribs in the ROI, based on an auto rib extracting algorithm; and
displaying, by the display unit, information about the measured number of ribs.

6. The method of claim 5, wherein the measuring comprises:
enhancing, by the measuring unit, a rib image in the ROI;
generating, by the measuring unit, a binary image by extracting an area having a brightness greater than or equal to a reference value from the enhanced rib image;
classifying, by the measuring unit, discrete areas from the binary image based on a level of an area;
connecting, by the measuring unit, the classified discrete areas along a principle axis to generate rib areas; and
counting, by the measuring unit, a number of the rib areas to measure the information about the number of ribs.

7. The method of claim 6, wherein the enhancing comprises:
enhancing, by the measuring unit, a picture quality or a definition of the rib image through a rib enhancement filtering process for segmentation of an ROI image.

8. The method of claim 5, wherein the displaying comprises:
displaying, by the display unit, the measured rib area and the information about the number of ribs?.

9. A computer readable recoding medium storing a program implementing the method of claim 5.
